(19) Europäisches Patentamt
European Patent Office
Office européen des brevets

(11) Publication number : **0 375 614 B1**

# (12) EUROPEAN PATENT SPECIFICATION

(45) Date of publication of patent specification : **31.03.93 Bulletin 93/13**

(51) Int. Cl.$^5$ : **C09D 5/08**

(21) Application number : **89810947.5**

(22) Date of filing : **12.12.89**

## (54) Corrosion-inhibiting coating compositions.

(30) Priority : **21.12.88 GB 8829828**
**08.02.89 GB 8902738**

(43) Date of publication of application : **27.06.90 Bulletin 90/26**

(45) Publication of the grant of the patent : **31.03.93 Bulletin 93/13**

(84) Designated Contracting States : **BE CH DE FR GB IT LI NL SE**

(56) References cited :
**EP-A- 0 129 506**
**DATABASE WPIL, no. 89-027 847 DERWENT PUBLICATIONS LTD., London, GB**

(73) Proprietor : **CIBA-GEIGY AG**
**Klybeckstrasse 141**
**CH-4002 Basel (CH)**

(72) Inventor : **Phillips, Emyr, Dr.**
**1 Hill Top View Tingley**
**Wakefield West Yorkshire (GB)**
Inventor : **Braig, Adalbert, Dr.**
**Zollstrasse 1/1**
**W-7858 Weil-Friedlingen (DE)**

(74) Representative : **Sharman, Thomas et al**
**CIBA-GEIGY PLC. Patent Department, Central Research, Hulley Road**
**Macclesfield, Cheshire SK10 2NX (GB)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to coating compositions, in particular to coating compostions containing, as corrosion inhibitors, amine salts of heterocyclic carboxylic acids.

Protection against corrosion is one of the most important functions of organic coating compositions for metal substrates. Many suggestions for improving the protection of coatings against corrosion are to be found in the literature, for example in H. Kittel, Lehrbuch der Lacke und Beschichtungen ("Textbook of Paints and Coatings"), Volume V, Stuttgart 1977,46-103.

On the one hand, the barrier function of the coating composition can be improved, in order to keep corrosive agents, such as oxygen, water and ions, away from the metal surface. On the other hand, it is possible to employ corrosion-inhibiting pigments which intervene chemically or electrochemically in the corrision process, for example by the formation of insoluble deposits with corrosion products or by passivation (polarisation) of the metal surface. Metal chromates and lead compounds rank amongst the most effective corrosion-inhibiting pigments. Much use has been made of metal chromates, particularly because they inhibit both anodic and cathodic corrosion. Nowadays there are certain objections to the use of chromates owing to their potential carcinogenic action. Similarly, there are objections to the use of lead compounds owing to their chronic toxicity.

Metal salts of organic compounds have also been frequently suggested as corrosion inhibitors. Thus, for example, European Patent Specification 3,187 recommends the use of zinc or lead salts of hydroxy or mercapto compounds of 5-membered or 6-membered heterocyclic compounds containing the characteristic group

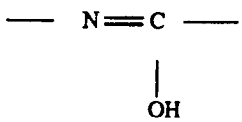

or

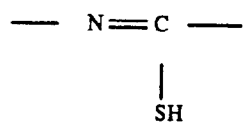

Typical examples of these are the Zn or Pb salts of 2-mercaptobenzthiazole.

More recently, in European Patent Application 128862 there have been described corrosion-inhibiting coating compositions containing:

a) a film-former;

b) as the corrosion inhibitor, an effective amount of an aliphatic or cycloaliphatic mono-, di-, tri- or tetracarboxylic acid which is subsituted in its aliphatic or cycloaliphatic radical by at least one group of the formula

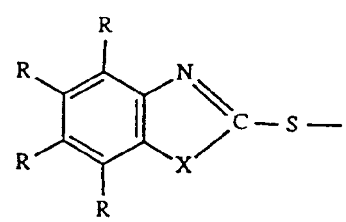

in which X is oxygen, sulfur or NH and each R independently of the others is hydrogen, alkyl, halogenoalkyl, alkoxy, alkylthio, alkylsulfonyl, cycloalkyl, phenyl, alkylphenyl, phenylalkyl, halogen, -CN, $-NO_2$, -COOH, COOalkyl, -OH or a primary, secondary or tertiary amino or carbamoyl group, R not being $-NH_2$, in the case of a monocarboxylic acid in which X is sulfur, and also base addition salts of these compounds.

The coating compositions of EP 128862 provide outstanding corrosion protection when the film former, component a) does not contain an acid-sensitive component.

When, however, the coating compositions of EP 128862 comprise as film-forming component a), an acid-sensitive component, e.g. when the film former a) is an epoxy resin, a polyurethane resin or it contains a basic binder material, unacceptable viscosity changes and/or coagulation and/or discolouration may occur in the coating composition.

JP-A-63301271 describes certain water-soluble compositions in which the corrosion inhibitor component is a $C_1$-$C_8$ alkylamine salt of benzothiazol-2-yl-thiosuccinic acid. There is no suggestion in this reference that the performance of the corrosion inhibitor is dependent on the acid-sensitivity of the film former used.

We have found that these potential problems with certain film formers may be overcome by employing an amine salt of the aliphatic or cycloaliphatic mono-, di-, tri- or tetracarboxylic acid as defined in EP 128862.

Accordingly, the present invention provides a corrosion-inhibiting coating composition comprising:

a) an acid-sensitive film former and

b) as a corrosion inhibitor, an effective amount of a salt of

i) an aliphatic or cycloaliphatic mono-, di-, tri- or tetra-carboxylic acid which is substituted in its aliphatic or cycloaliphatic radical by at least one group of the formula

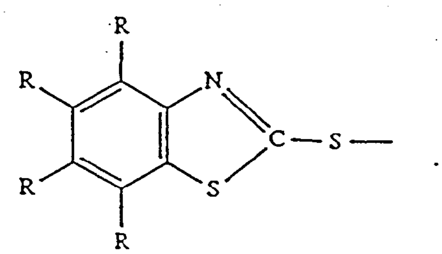

I

in which each R independently of the others is hydrogen, alkyl, halogenoalkyl, alkoxy, alkylthio, alkylsulfonyl, cycloalkyl, phenyl, alkylphenyl, phenylalkyl, halogen, -CN, $-NO_2$, -COOH, -COOalkyl, -OH or a primary, secondary or tertiary amino or carbamoyl group;

with

ii) n molar equivalents of amine in which n is 1, 2, 3 or 4 depending upon the number of carboxyl groups in the carboxylic acid b i), characterised in that the amine b ii) has the formula:

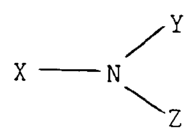

in which X is $C_{10}$-$C_{24}$ unsubstituted alkyl or $C_2$-$C_{24}$ alkyl interrupted by one or more oxygen atoms, and Y and Z are the same or different and each is hydrogen, alkyl optionally interrupted by one or more oxygen atoms, or alkyl substituted by hydroxy, or are phenyl, phenylalkyl or alkylphenyl.

As alkyl, alkoxy, alkylthio or alkylsulfonyl, R preferably contains 1-12 C atoms, especially 1-6 C atoms. Examples of these are methyl, ethyl, propyl, isopropyl, n-butyl, isobutyl, tert.-butyl, pentyl, hexyl, octyl, nonyl, decyl, undecyl, dodecyl and the corresponding alkoxy, alkylthio and alkylsulfonyl radicals. As cycloalkyl, R preferably contains 5-8 C atoms. Examples of these are cyclopentyl, cyclohexyl or cyclooctyl.

As halogenalkyl, R preferably contains 1-4 C atoms and 1-3 fluorine or chlorine atoms. Examples of these are chloromethyl, fluoromethyl, difluoromethyl, trifluoromethyl or 2-chloroethyl.

As alkylphenyl, R preferably contains 7-16 C atoms and can be for example, tolyl, xylyl, 4-isopropylphenyl, 4-tert.-butylphenyl, 4-octylphenyl or 4-decylphenyl. As phenylalkyl, R preferably contains 7-9 C atoms and can be, for example, benzyl, 1-phenylethyl, 2-phenylethyl, $\alpha,\alpha$-dimethylbenzyl or 3-phenylpropyl.

As halogen, R is preferably fluorine, chlorine or bromine. If R is -COOalkyl, the alkyl group preferably has 1-4 C atoms.

As an amino group or carbamoyl group, R preferably has up to 20 C atoms. Examples of these are groups $-NH_2$, $-NHCH_3$, $-NHC_{12}H_{15}$, -NH-cyclohexyl, -NH-phenyl, $-N(CH_3)_2$, $-N(C_4H_9)_2$, $-N(CH_3)$(benzyl), morpholino, piperidino, $-CONH_2$, -CONHphenyl, $-CONHC_8H_{17}$, $-CON(C_2H_5)_2$, $-CON(CH_2CH_2OH)_2$, morpholinocarbonyl or piperidinocarbonyl.

Preferably, one of the substituents R is hydrogen, $C_1$-$C_4$-alkyl, $C_1$-$C_4$-alkoxy and the other three R are hydrogen. It is particularly preferably for all four R to be hydrogen.

As alkyl , Y and Z preferably contain 1-24 C atoms, especially 6-24 C atoms, more especially 8-14 C atoms. Examples of alkyl groups Y and Z are methyl, ethyl, propyl, isopropyl, n-butyl, isobutyl, tert.-butyl, pentyl, hexyl, octyl and nonyl, and examples of alkyl groups X, Y and Z are decyl, dodecyl, tetradecyl, hexadecyl, octadecyl, eicosyl, and tetraeicosyl radicals. Alkyl radicals X, Y and Z interrupted by one or more oxygen atoms include methoxymethyl, 1-methoxyethyl, 2-ethoxypropyl, 1-methoxybutyl, n-butoxymethyl and 4-isopropoxybutyl. Alkyl radicals Y and Z substituted by hydroxy are e.g. hydroxymethyl, 1-hydroxyethyl, 1-hydroxypropyl, 2-hydroxypropyl and 1-hydroxybutyl. As phenylalkyl, Y and Z preferably contain 7-9 C atoms and may be, e.g., benzyl, 1-phenylethyl, 2-phenylethyl, $\alpha,\alpha$-dimethylbenzyl or 3-phenylpropyl.

The component bi) is preferably a monocarboxylic or dicarboxylic acid, in particular a dicarboxylic acid. The substituent of the formula I is preferably in the beta-position in relation to a carboxyl group.

The component bi) is preferably an aliphatic monocarboxylic or polycarboxylic acid which has 2-20 C atoms or a cycloaliphatic monocarboxylic or polycarboxylic acid which has 4-12, in particular 6-8, C atoms and which is substituted by a group of the formula I.

In addition to the group of the formula I, the carboxylic acid can also have other substituents, for example hydroxyl, alkoxy, halogen or aryl.

Components bi) which are preferred are compounds of the formula II

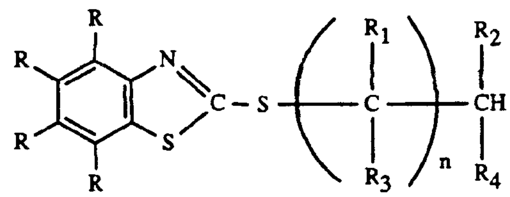

II

in which R has the meaning given above, n is zero or one and $R_1$, $R_2$, $R_3$ and $R_4$ independently of one another are hydrogen, alkyl, hydroxyalkyl, halogenalkyl, alkoxyalkyl, carboxyalkyl, carboxyl or phenyl or phenylalkyl which is unsubstituted or monosubstituted or disubstituted, or $R_1$ and $R_2$ or $R_1$ and $R_3$ together are linear or branched alkylene which can be substituted by 1 or 2 carboxyl groups, or $R_1$ and $R_2$ together are a direct bond, and at least one of the substituents $R_1$, $R_2$, $R_3$ and $R_4$ is a carboxyl or carboxyalkyl group. In formula II, n is preferably one.

As alkyl, $R_1$, $R_2$, $R_3$ and $R_4$ are preferably $C_1$-$C_{18}$-alkyl, for example methyl, ethyl, n-propyl, isopropyl, n-butyl, isobutyl, tert.-butyl, pentyl, hexyl, octyl, dodecyl or octadecyl. As hydroxyalkyl or halogenoalkyl, these substituents preferably have 1-4C atoms. Examples of these are hydroxymethyl, 1-hydroxyethyl, 2-hydroxyethyl, 2-hydroxypropyl, 3-hydroxypropyl, chloromethyl, bromoethyl or bromoisopropyl. As alkoxyalkyl, these substituents preferably have 2-10 C atoms. Examples of these are methoxymethyl, 1-methoxyethyl, 2-ethoxypropyl, 1-methoxybutyl, n-butoxymethyl or 4-isopropoxybutyl.

As carboxyalkyl, $R_1$, $R_2$, $R_3$ and $R_4$ are preferably $C_2$-$C_{12}$-carboxyalkyl, for example carboxymethyl, 1-carboxyethyl, 2-carboxyethyl, 3-carboxypropyl, 2-carboxyisopropyl, 1-carboxybutyl, 2-carboxybutyl, 1-, 2- or 3-carboxyhexyl, 1,2-dicarboxyethyl or 2,3,4-tricarboxyl-1-butyl. As substituted or unsubstitued phenyl or phenylalkyl, the same substituents can be, for example, 4-chlorophenyl, 3-nitrophenyl, tolyl, xylyl, 3-methoxyphenyl, 4-isopropylphenyl, 3-carboxyethyl, 4-hydroxyphenyl, 4-bromobenzyl, 4-tert.-butylbenzyl, 2-phenylethyl or 3-phenylpropyl, but are preferably phenyl or benzyl.

If $R_1$ and $R_2$ or $R_1$ and $R_3$ together are alkylene, they are preferably $C_3$-$C_4$-alkylene and they form, together with the C atoms to which they are linked, a cycloalkane ring, preferably a cyclopentane or cyclohexane ring, which can be substituted by alkyl groups, preferably $C_1$-$C_4$-alkyl groups, or by 1 or 2 carboxyl groups.

If $R_1$ and $R_2$ together are a direct bond, the compounds of the formula II are unsaturated carboxylic acids.

$R_1$, $R_2$, $R_3$ and $R_4$ are preferably hydrogen, $C_1$-$C_4$-alkyl, carboxyl or $C_2$-$C_6$-carboxyalkyl. It is particularly preferable for $R_4$ to be a carboxyl group. Compounds of the formula II in which at least two of the substituents $R_1$ $R_2$, $R_3$ and $R_4$ are a carboxyl or carboxyalkyl group are also preferred.

The following are examples of the component bi)

benzothiazol-2-ylthioacetic acid,
5-carboxybenzothiazol-2-ylthioacetic acid,
3-(benzothiazol-2-ylthio)-propionic acid,
5-trifluoromethylbenzothiazol-2-ylthiopropionic acid,
4-(benzothiazol-2-ylthio)-butyric acid,
3-(benzothiazol-2-ylthio)-butyric acid,
3-(benzothiazol-2-ylthio)-methylbutyric acid,
benzothiazol-2-ylthiomalonic acid,
benzothiazol-2-ylthiosuccinic acid,
5-methylbenzothiazol-2-ylthiosuccinic acid,
6-ethylbenzothiazol-2-ylthiosuccinic acid,
4-isopropylbenzothiazol-2-ylthiosuccinic acid,
7-t-butylbenzothiazol-2-ylthiosuccinic acid,
5-n-hexylbenzothiazol-2-ylthiosuccinic acid,
6-(1,1,3,3-tetramethylbutyl)-benzothiazol-2-ylthiosuccinic acid,
6-cyclohexylbenzothiazol-2-ylthiosuccinic acid,
7-benzylbenzothiazol-2-ylthiosuccinic acid,

6-methoxybenzothiazol-2-ylthiosuccinic acid,
6-ethoxybenzothiazol-2-ylthiosuccinic acid,
7-ethoxybenzothiazol-2-ylthiosuccinic acid,
5-methoxybenzothiazol-2-ylthiosuccinic acid,
4-methylthiobenzothiazol-2-ylthiosuccinic acid,
4-fluorobenzothiazol-2-ylthiosuccinic acid,
5-chlorobenzothiazol-2-ylthiosuccinic acid,
7-bromobenzothiazol-2-ylthiosuccinic acid,
6-chlorobenzothiazol-2-ylthiosuccinic acid,
4-phenylbenzothiazol-2-ylthiosuccinic acid,
5-trifluoromethylbenzothiazol-2-ylthiosuccinic acid,
5-carboxybenzothiazol-2-ylthiosuccinic acid,
6-methylsulfonylbenzothiazol-2-ylthiosuccinic acid,
5-cyanobenzothiazol-2-ylthiosuccinic acid,
6-nitrobenzothiazol-2-ylthiosuccinic acid,
5-cyanobenzothiazol-2-ylthiosuccinic acid,
7-hydroxybenzothiazol-2-ylthiosuccinic acid,
6-chloro-4-methylbenzothiazol-2-ylthiosuccinic acid,
5-chloro-6-n-butylbenzothiazol-2-ylthiosuccinic acid,
4-bromo-5-n-hexylbenzothiazol-2-ylthiosuccinic acid,
5-nitro-6-n-propylbenzothiazol-2-ylthiosuccinic acid,
5-bromo-6-n-propoxybenzothiazol-2-ylthiosuccinic acid,
6-aminobenzothiazol-2-ylthiosuccinic acid,
6-methylaminobenzothiazol-2-ylthiosuccinic acid,
5-dimethylaminobenzothiazol-2-ylthiosuccinic acid,
7-phenylaminobenzothiazol-2-ylthiosuccinic acid,
6-diphenylaminobenzothiazol-2-ylthiosuccinic acid,
4-benzylaminobenzothiazol-2-ylthiosuccinic acid,
4-morpholinobenzothiazol-2-ylthiosuccinic acid,
5-carbamoylbenzothiazol-2-ylthiosuccinic acid,
5-methylcarbamoylbenzothiazol-2-ylthiosuccinic acid,
5-diethylcarbamoylbenzothiazol-2-ylthiosuccinic acid,
6-phenylcarbamoylbenzothiazol-2-ylthiosuccinic acid,
5,6-dimethylbenzothiazol-2-ylthiosuccinic acid,
4,5,6-triethylbenzothiazol-2-ylthiosuccinic acid,
4,5,6,7-tetramethylbenzothiazol-2-ylthiosuccinic acid,
1-(benzothiazol-2-ylthio)-propane-1,2-dicarboxylic acid,
3-(benzothiazol-2-ylthio)-propane-1,2-dicarboxylic acid,
3-(6-trifluoromethylbenzothiazol-2-ylthio)-propane-1,2-dicarboxylic acid,
3-(6-methoxycarbonylbenzothiazol-2-ylthio)-propane-1,2-dicarboxylic acid,
3-(6-aminobenzothiazol-2-ylthio)-propane-1,2-dicarboxylic acid,
3-(5-ethylaminobenzothiazol-2-ylthio)-propane-1,2-dicarboxylic acid,
3-(4-dibutylaminobenzothiazol-2-ylthio)-propane-1,2-dicarboxylic acid,
4-(morpholinobenzothiazol-2-ylthio)-propane-1,2-dicarboxylic acid,
1-(4-phenylbenzothiazol-2-ylthio)-propane-1,2-dicarboxylic acid,
1-(benzothiazol-2-ylthio)-propane-1,3-dicarboxylic acid,
1-(6-ethylbenzothiazol-2-ylthio)-propane-1,3-dicarboxylic acid,
2-(benzothiazol-2-ylthio)-propane-1,3-dicarboxylic acid,
2-(5-carboxybenzothiazol-2-ylthio)propane-1,3-dicarboxylic acid,
3-(benzothiazol-2-ylthio)-3-phenylpropane-1,2-dicarboxylic acid,
3-(benzothiazol-2-ylthio)-3-(4-carboxyphenyl)-propane-1,2-dicarboxylic acid,
3-(benzothiazol-2-ylthio)-3-(2,4-dicarboxyphenyl)-propane-1,2-dicarboxylic acid,
3-(benzothiazol-2-ylthio)-3,3-diphenylpropane-1,2-dicarboxylic acid,
1-(benzothiazol-2-ylthio)-butane-1,2-dicarboxylic acid,
1-(4-methoxy-6-hydroxybenzothiazol-2-ylthio)-butane-1,2-dicarboxylic acid,
3-(4,5-dimethyl-7-propoxybenzothiazol-2-ylthio)-propane-1,2-dicarboxylic acid,
1-(benzothiazol-2-ylthio)-2-methylpropane-1,2-dicarboxylic acid,
2-(benzothiazol-2-ylthio)-butane-2,3-dicarboxylic acid,

1-(benzothiazol-2-ylthio)-butane-2,4-dicarboxylic acid,
4-(benzothiazol-2-ylthio)-butane-1,2,3-tricarboxylic acid,
4-(benzothiazol-2-ylthio)-butane-1,4-dicarboxylic acid,
1-(benzothiazol-2-ylthio)-pentane-1,5-dicarboxylic acid,
3-(benzothiazol-2-ylthio)-hexane-1,2-dicarboxylic acid,
8-(benzothiazol-2-ylthio)-octane-1,3,5,7-tetracarboxylic acid,
1-(benzothiazol-2-ylthio)-cyclohexane-1,2-dicarboxylic acid,
4-(benzothiazol-2-ylthio)-cyclohexane-1,2-dicarboxylic acid,
1-(benzothiazol-2-ylthio)-propane-1,2,3-tricarboxylic acid,
1-(benzothiazol-2-ylthio)-3-chloropropane-1,2-dicarboxylic acid,
1-(benzothiazol-2-ylthio)-3-methoxypropane-1,2-dicarboxylic acid,
1-(benzothiazol-2-ylthio)-3-hydroxypropane-1,2-dicarboxylic acid,
1-(benzothiazol-2-ylthio)-2-phenylsuccinic acid,
1-(benzothiazol-2-ylthio)-2-benzylsuccinic acid,
1-(benzothiazol-2-ylthio)-3-methylbutane-1,3-dicarboxylic acid,
3-(benzothiazol-2-ylthio)-hexane-3,4-dicarboxylic acid,
2,3-bis-(benzothiazol-2-ylthio)-butane-1,4-dicarboxylic acid,
and mixtures thereof.

The following are examples of the component bii):

n-decylamine
n-dodecylamine
iso-dodecylamine
t-dodecylamine
n-tridecylamine
iso-tridecylamine
t-tridecylamine
n-tetradecylamine
iso-tetradecylamine
t-tetradecylamine
n-octadecylamine
iso-octadecylamine
t-octadecylamine
n-nonadecylamine
iso-nonadecylamine
t-nonadecylamine
n-eicosamine
iso-eicosamine
t-eicosamine
n-heneicosamine
iso-heneicosamine
t-heneicosamine
n-docosamine
iso-docosamine
t-docosamine
n-tricosamine
iso-tricosamine
t-tricosamine
n-tetracosamine
iso-tetracosamine
t-tetracosamine
di-n-dodecylamine
di-n-eicosylamine
di-n-tetraeicosylamine
methoxymethylamine
methoxyethylamine
butoxypropylamine
hexoxybutylamine
nonyloxypropylamine

and mixtures thereof.

The preparation of the compounds can be effected in accordance with a process described in European Patent Application 129506, by reacting a compound of the formula II

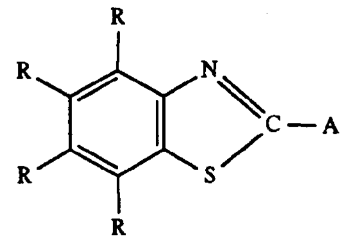

III

in which A is a leaving group, for example Cl, Br, I or p-tosyloxy, with a compound of the formula

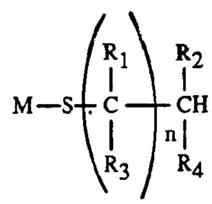

in which M is hydrogen or a cation, for example an alkali metal cation, alkaline earth metal cation or ammonium cation. Alternatively, a compound of the formula IV

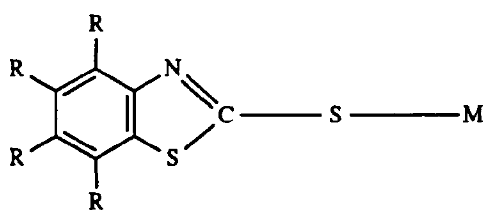

IV

can be reacted with a compound of the formula

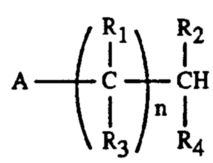

Compounds of the formula II in which $R_4$ is carboxyl can also be prepared by reacting IV, in which M is hydrogen, with an α,ß-unsaturated acid of the formula

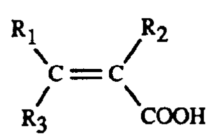

in accordance with a process which is described in US Patent 4612378

Salts may be prepared from components bi) and bii) by heating together the said components at 30-130°C preferably at 50-60°C, optionally in a solvent e.g. methanol, xylene, or tetrahydrofuran.

Those salts in which, in the amine of formula II, X is as defined hereinbefore and Y and Z, independently, are hydrogen, $C_1$-$C_{24}$ unsubstituted alkyl or $C_2$- $C_{24}$ alkyl interrupted by one or more oxygen atoms or substituted by hydroxy, or are phenyl or $C_7$-$C_9$ phenylalkyl, are new compounds and, as such, form a further aspect of the present invention.

The component a) can be any desired acid-sensitive film-former, such as those which are knwon as binders for coating compositions. In particular, it can be an epoxide resin, polyurethane resin, aminoplast resin, or a mixture of such resins or a basic aqueous dispersion or solution of an acidic resin. The film-forming component a) may be a solution of the binder resin in an organic solvent, it may be an aqueous solution or dispersion or it may be a solid powder. Of special industrial importance are "high solids coatings" containing a limited amount of organic solvent. Suitable epoxide resins are those which have an average more than one epoxide group per molecule, for example bis-(2,3-epoxypropyl-cyclohexyl) ether, 4-epoxyethylcyclohexene oxide or the 2-methyl-4,5-epoxycyclohexylmethyl ester of 2-methyl-4,5-epoxycyclohexanecarboxylic acid; diglycidyl and polyglycidyl esters of aliphatic polyols, for example 1,4-butanediol or polyalkylene glycols; diglycidyl or polyglycidyl ethers of cycloaliphatic polyols, for example 2,2-bis-(4-hydroxycyclohexyl)-propane; diglycidyl and polyglycidyl ethers of aromatic polyols, for example resorcinol, bis-(4-hydroxyphenyl)-methane, 2,2-bis-(4-hydroxyphenyl)-propane, 2,2-bis-(4-hydroxy-3,5-dibromophenyl)-propane, 1, 1,2,2-tetrakis-(4-hydroxyphenyl)-ethane or condensation products of phenols with formaldehyde, such as phenol or cresol novolacs; β-methylglycidyl ethers of polyols; glycidyl esters of polybasic carboxylic acids, for example phthalic, tetraphthalic, tetrahydrophthalic or hexahydrophthalic acid; N-glycidyl derivatives or amines, amides or nitrogen-heterocyclic compounds, for example N,N-diglycidylaniline, N,N-diglycidyltoluidine or N,N,N′,N′-tetraglycidyl-bis-(4-aminophenyl)-methane, triglycidyl isocyanurate, N,N′-diglycidylethyleneurea, N,N′-diglycidyl-5,5-diethylhydantoin; N,N′-diglycidyl-5-isopropylhydantoin or N,N′-diglycidyl-5,5-dimethyl-6-isopropyl-5,6-dihydrouracil.

Preferred epoxide resins are those based on aromatic polyols, in particular bisphenols. The epoxide resins are used in conjunction with a curing agent. The latter can be, in particular, an amino or hydroxy compound or an acid or an acid anhydride or a Lewis acid. Examples of these are polyamines, polyaminoamides, polysulfide polymers, polyphenols, boron fluoride and complexes thereof, polycarboxylic acids, 1,2-dicarboxylic acid anhydrides or pyromellitic dianhydride.

In addition to the components a) and b), the coating compositions can also contains further components, for example pigments, dyes, extenders and other additives such as are customary for coating compositions. The pigments can be organic, inorganic or metallic pigments, for example titanium dioxide, iron oxide, aluminium bronze, phthalocyanine blue etc. It is also possible to use concomitantly anti-corrosion pigments, for example pigments containing phosphates or borates, metal pigments and metal oxide pigments (see Farbe und Lack 88(1982), 183) or the pigments descirbed in European Patent A 54,267. Examples of extenders which can be used concomitantly are talc, chalk, alumina, baryte, mica or silica. Examples or further additives are flow control auxiliaries, dispersing agents, thioxotropic agents, adhesion promoters, antioxidants, light stabilisers or curing catalysts.

Particular importance attaches the addition of basic extenders or pigments. In certain binder systems, for example in acrylic and alkyd resins, these produce a synergistic effect on the inhibition of corrosion. Examples or such basic extenders or pigments are calcium carbonate, magnesium carbonate, zinc oxide, zinc carbonate, zinc phosphate, magnesium oxide, aluminium oxide, aluminium phosphate or mixtures thereof. Examples of pigments are those based on aminoanthraquinone.

Finally, the corrosion inhibitor can also be applied to a neutral carrier. Suitable carriers are, in particular, pulverulent extenders or pigments. This technique is described in greater detail in German Offenlegungsschrift 3,122,907.

In addition to the component b), the coating composition can also contain another organic, metal-organic or inorganic corrosion inhibitor, for example salts of nitroisophthalic acid, tannin, phosphoric esters, technical amines, substituted benztriazoles or substituted phenols, such as are described in German Offenlegungsschrift 3,146,265.

The coating compositions according to the invention are preferably used as a primer on metallic substrates, in particular on iron, steel, copper, zinc and aluminium. Here they can function as so-called conversion coatings, in that chemical reactions take place at the interface between the metal and the coating. The application of the coatings can be effected by the customary methods, such as spraying, brushing, roller-coating, dipping or electrodeposition, in particular cathodic deposition. Depending on whether the film-former is a resin which dries physically or can be cured by heat or radiation, the curing of the coatings is carried out at room temperature, by stoving or by irradiation.

The corrosion inhibitors can be added to the coating composition during the preparation of the latter, for example during the distribution of the pigment by grinding or the inhibitors are dissolved beforehand in a solvent and the solution is stirred into the coating composition. The inhibitor is used in an amount of 0.01-20 % by weight, preferably 0.1-5 % by weight, based on the solids content of the coating composition.

The following Examples describe the coating compositions according to the invention and their use in greater detail.

Example 1: 11.9 Parts of benzothiazol-2-ylthiosuccinic acid, suspended in 28.7 parts xylene, are treated

with 16.8 parts of t-tridecylamine. The resulting slurry is heated to 60°C to give a pale yellow solution. Evaporation of this solution gives 39 parts bis (t-tridecylammonium) benzothiazol-2-ylthiosuccinate as a viscous yellow oil.

Potentiometric analysis shows the salt to contain 42.4 % benzothiazol-2-ylthiosuccinic acid and 57.6 % t-tridecylamine, a molar ratio of 1:2.03.

Example 2: 160.8 Parts of i-nonyloxypropylamine are added to a solution of 113.2 parts benzothiazol-2-ylthiosuccinic acid, in 100 parts tetrahydrofuran. Evaporation of this solution gives 274 parts bis (isononyloxypropylammonium) benzothiazol-2-ylthio-succinate as a brown oil.

Potentiometric titration shows the salt to contain 39.75 % benzothiazol-2-ylthiosuccinic acid and 60.25 % isononyloxypropylamine, a molar ratio of 1:2.

Example 3: 104.3 Parts of t-eicosylamine and 99 parts of benzothiazol-2-ylthiosuccinic acid are mixed and warmed to 50°C to give 203 parts bis(t-eicosylammonium) benzothiazol-2-ylthiosuccinate.

Potentiometric analysis shows the salt to contain 34.5 % benzothiazol-2-ylthiosuccinic acid and 65.5 % t-eicosylamine, a molar ratio of 1:2.

Examples 4 to 6: A two-pack epoxy primer is prepared using the following formulation:

16.0 parts wt. of Araldite GT 6071 (Araldite is a registered Trade Mark) as a 75 % solution in a mixture of toluene and n-butanol;

37.1 parts wt. of red iron oxide;
20.1 parts wt. of micronised talc;
16.1 parts wt. of propyleneglycol monomethyl ether;
6.9 parts wt. of isopropanol;
3.2 parts wt. of Solvesso 100 and
0.6 part wt. of soya lecithin.
1.88 pts. wt. (3 %) of a product of Examples 1 to 3.

is dispersed into a separate sample of the epoxy part, on a ball mill. The concentration of corrosion inhibitor of this invention is based on the solids content of the total system (including polyaminoamide hardener). The solids content of the formulation is 62.6 %, calculated for 100 g of finished paint including hardener.

After the dispersion procedure, 50 pts. wt. of polyaminoamide hardener (HY 815 as a 50% solution) are added.

The viscosity properties of the paints so obtained are set out in the following Table I:

Table I

| Example | Additive | % Add. | Initial viscosity (poise) | Viscosity after 30 mins (poise) |
|---|---|---|---|---|
| - | Control | nil | 2.9 | 3.0 |
| 4 | Product Ex.1 | 3 % | 3.3 | 3.2 |
| 5 | Product Ex.2 | 3 % | 2.2 | 2.9 |
| 6 | Product Ex.3 | 3 % | 3.1 | 3.0 |

It can be seen that incorporation of an amine salt corrosion inhibitor of the invention of the invention causes no significant viscosity change. When used at the same level of addition the free carboxylic acid counterpart of the amine salts of Examples 1 to 3 causes an unacceptable viscosity increase.

The respective paints so obtained are then applied on to cold rolled steel plates (10 x 15 cm) at a dry film thickness of 75 microns. The films are then cured for 7 days at 20°C. A white polyurethane topcoat is then applied and cured at 80° for 45 minutes.

The cured paint surface is scribed (7 x 0.05 cm) until the metal is reached, using bonder cross-cut device. An edge protection agent (Icosit® 255) is applied to the edges in order to protect them.

The samples are now subjected to a salt spray test as specified in ASTM B 117 for a duration of 1000 hours. The condition of the coating is assessed after every 200 hours of weathering, specifically the degree of blistering (as specified in DIN 53,209) at the cross-cut and on the painted surface and also the degree of rusting (as specified in DIN 53,210) on the entire surface.

At the end of the test, the coating is removed, and the corrosion of the metal at the cross-cut (as specified in DIN 53,167) and also over the remainder of the surface is assessed. In every case the assessment is made on the basis of a 6-stage scale. The corrosion protection value CP is given by the sum of the assessment of the coating and the assessment of the metal surface. The higher this value, the more effective the inhibitor under test.

The results are set out in Table II.

Table II

| Example | Additive | % Additive | Assessment of coating | Assessment of metal | CP |
|---|---|---|---|---|---|
| - | Control | nil | 2.6 | 4.3 | 6.9 |
| 4 | Product Ex.1 | 1.0 | 4.0 | 4.3 | 8.3 |
| 5 | Product Ex. 2 | 1.0 | 4.0 | 4.6 | 8.6 |
| 6 | Product Ex 3 | 1.0 | 4.0 | 4.0 | 8.0 |

Examples 7 to 9: An epoxy ester paint having 50 % solids content is prepared using the following formulation:

| | |
|---|---|
| 32.90 % | wt. Duroxyn® EF 900 (60 % in xylene), an epoxy resin ester supplied by Hoechst A.G. |
| 2.24 % | wt. red iron oxide |
| 4.48 % | wt. micronised talc |
| 11.22 % | wt. barium sulphate |
| 1.49 % | wt. aluminium silicate |
| 10.46 % | wt. titanium dioxide |
| 0.29 % | wt. anti-skinning agent |
| 0.10 % | wt. cobalt naphthenate (8 %) |
| 36. 82 % | wt. 4:1 mixture of white spirit/aromatic. |

1.5 g (3 % on total solids content) of a product of Examples 1 to 3 is dispersed into separate samples of the paint so formulated.

Each paint sample is then applied on to cold roll steel plates (10 x 15 cm) at a dry film thickness of 55-60 microns. The films are cured at 20°C for 7 days.

The respective plates are then scribed and subjected to the salt spray test procedure (600 hours) described in Examples 4 to 6. The results are set out in Table III:

Table III

| Example | Additive | % Additive | Assessment of coating | Assessment of metal | CP |
|---|---|---|---|---|---|
| - | Control | nil | 4.2 | 2.5 | 6.7 |
| 7 | Product Ex.1 | 3 | 4.2 | 4.9 | 9.1 |
| 8 | Product Ex.2 | 3 | 4.0 | 4.4 | 8.4 |
| 9 | Product Ex.3 | 3 | 4.4 | 4.8 | 9.2 |

Examples 10 to 12:An aqueous alkaline paint formulation having a solids content of 56,15 wt % is prepared using the following formulation:

60.03 wt % Bayhydrol® B 30 (30 % in water), an aqueous alkyd resin supplied for Bayer A.G.
0.14 wt % Servosyn® WEB (8 %), a siccative (Servo B.B.)
0.28 wt % Ascinin® R
21.13 wt % Bayferrox® 130M, an iron red oxide (Bayer AG)
5.15 wt % Heladol® 10 (calcium carbonate)
10.6 wt % micronised talc
0.2 wt % Aerosil® 300 (a thixotropic agent ex Degussa)
1.06 wt % ZnO
0.9 wt % butylglycol
0.05 wt % aluminium octoate
0.46 wt % water.
0.56 wt % (1 % by weight on solids content) of a product of Examples 1 to 3 is dispersed in separate samples of the paint formulation.

Each paint sample is applied on to cold roll steel plates at a layer thickness of 55-60 microns, and dried for 72 hours at 20°C. The painted plates are then placed in a sealed chamber and exposed for 700 hours to condensed moisture at 40°C/100 % relative humidity. The results are summarised in the following Table IV

Table IV

| Example | Additive | % Additive | Assessment of coating | Assessment of metal | CP |
|---|---|---|---|---|---|
| - | Control | nil | 5.4 | 1.7 | 7.1 |
| 10 | Product Ex.1 | 1 | 5.8 | 5.0 | 10.8 |
| 11 | Product Ex. 2 | 1 | 6.0 | 5.0 | 11.0 |
| 12 | Product Ex. 3 | 1 | 5.4 | 4.9 | 10.3 |

Examples 13 to 15:A two-pack polyurethane primer is prepared according to the following formulations:

57.9 wt % Macrynal® 5M 510n (an acrylic resin containing hydroxyl groups, Hoechst A.G.)
0.3 wt % Aerosil® R972 (silicon anti-settling agent)

26.3 wt % titanium dioxide RN59
8.5 wt % butyl glycol acetate
0.07 wt % zinc octoate
4.03 wt % Solvesso® 100 (mixture of aromatic solvents ex Esso A.G.)
2.1 wt % methyl isobutyl ketone
0.2 wt % BYE 344 (gloss improver, Byk-Mallinckrodt)
0.6 wt % BYE 0 (an antifoam agent)

The above components are dispersed on a ball mill and then 23.3 g of Desmodur® N75 are added. The viscosity properties of the paints so obtained are set out in Table V

Table V

| Example | Additive | % Additive | Initial viscosity (poise) | Viscosity after 20 mins (poise) |
|---|---|---|---|---|
| - | Control | nil | 4.1 | 4.6 |
| 13 | Product Ex.1 | 2 % | 5.2 | 5.9 |
| 14 | Product Ex.2 | 2 % | 4.8 | 5.2 |
| 15 | Product Ex.3 | 2 % | 5.1 | 5.5 |

It can be seen that no significant viscosity increase is experienced with amine salts of the invention, in contrast with the parent free carboxylic acid which causes rapid increase in viscosity.

After dilution to spray viscosity, the respective paints are applied on to cold rolled steel plates and baked at 80°C/45 min. and then over-baked at 130°C/60 min. to ascertain any yellowing. The results are summarized in Table VI

Table VI

| Example | Additive | Yellowness Index after 80°C/45 min | 80°C/45 min + 130°C/60 min |
|---|---|---|---|
| - | Control | 3.3 | 3.4 |
| 13 | Product of Ex.1 | 4.2 | 5.5 |
| 14 | Product of Ex. 2 | 4.0 | 4.8 |
| 15 | Product of Ex. 3 | 4.1 | 5.6 |

Much more severe yellowing is noted when the amine salts of the invention are replaced by the parent carboxylic acid.

<u>Example 16</u>: 60 Parts of eicosylamine are added to a solution of 28.3 parts of benzothiazol-2-ylthiosuccinic

acid in 100 parts tetrahydrofuran. Evaporation of this solution gives 86.9 parts bis(eicosylammonium)benzothiazol-2-ylthiosuccinate as a brown oil having the following analysis C 69.6; H 10.87; N 5.06 %.

Example 17: An aqueous alkaline paint formulation is prepared as described in Examples 10 to 12.

The painted plates are placed in a sealed chamber and exposed for 800 hours to condensed moisture at 40°C/100% relative humidity.

The results are summarised in Table VII

Table VII

| Example | Additive | % Additive | Assessment of coating | Assessment of metal | CP |
|---|---|---|---|---|---|
| - | Control | nil | 5.4 | 1.9 | 7.1 |
| 17 | Product Ex. 16 | 2 | 5.9 | 1.7 | 7.6 |

## Claims

1. A corrosion-inhibiting coating composition comprising:
   a) an acid-sensitive film former and
   b) as a corrosion inhibitor, an effective amount of a salt of
   i) an aliphatic or cycloaliphatic mono-, di-, tri- or tetra-carboxylic acid which is substituted in its aliphatic or cycloaliphatic radical by at least one group of the formula

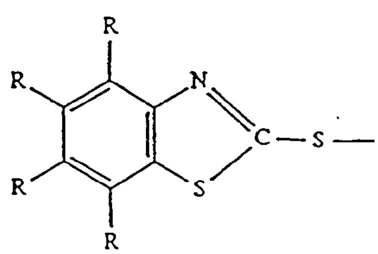

I

in which each R independently of the others is hydrogen, alkyl, halogenoalkyl, alkoxy, alkylthio, alkylsulfonyl, cycloalkyl, phenyl, alkylphenyl, phenylalkyl, halogen, -CN, $-NO_2$, -COOH, -COOalkyl, -OH or a primary, secondary or tertiary amino or carbamoyl group;
with
ii) n molar equivalents of an amine in which n is 1, 2, 3 or 4 depending upon the number of carboxyl groups in the carboxylic acid b i ), characterised in that the amine b ii) has the formula:

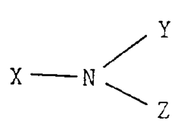

in which X is $C_{10}$-$C_{24}$ unsubstituted alkyl or $C_2$-$C_{24}$ alkyl interrupted by one or more oxygen atoms, and Y and Z are the same or different and each is hydrogen, alkyl optionally interrupted by one or more oxygen atoms, or alkyl substituted by hydroxy, or are phenyl, phenylalkyl or alkylphenyl.

2. A composition according to claim 1 wherein one of the substituents R is hydrogen, $C_1$-$C_4$ alkyl or $C_1$-$C_4$ alkoxy and the other three R are hydrogen.

3. A composition according to claim 2 wherein all four substituents R are hydrogen.

4. A composition according to claim 1 wherein component bi) is a compound of formula II

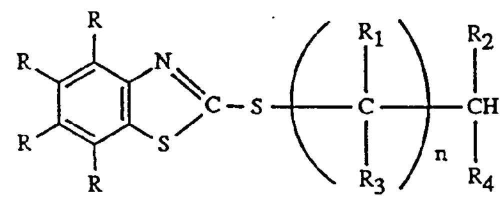

II

wherein R is as defined in claim 1, n is zero or one and $R_1$ $R_2$, $R_3$ and $R_4$ independently of one another are hydrogen, alkyl, hydroxyalkyl, halogenalkyl, alkoxyalkyl, carboxyalkyl, carboxyl or phenyl or phenylalkyl which is unsubstituted or monosubstituted or disubstituted, or $R_1$ and $R_2$ or $R_1$ and $R_3$ together are linear or branched alkylene which can be substituted by 1 or 2 carboxyl groups, or $R_1$ and $R_2$ together are a direct bond, and at least one of the substituents $R_1$, $R_2$, $R_3$ and $R_4$ is a carboxyl or carboxyalkyl group.

5. A composition according to claim 4 wherein n is 1.

6. A composition according to claim 4 wherein $R_4$ is a carboxyl group.

7. Salts of i) an aliphatic or cycloaliphatic mono-, di tri- or tetra-carboxylic acid which is substituted in its aliphatic or cycloaliphatic radical by at least one group of the formula

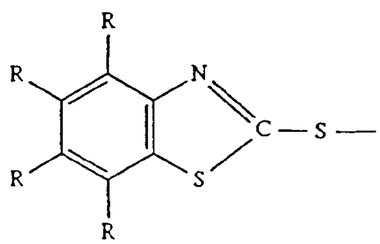

I

in which each R independently of the others is hydrogen, alkyl, halogenoalkyl, alkoxy, alkylthio, alkylsulfonyl, cycloalkyl, phenyl, alkylphenyl, phenylalkyl, halogen, -CN, $-NO_2$, -COOH, COOalkyl, -OH or a primary, secondary or tertiary amino or carbamoyl group; with ii) n molar equivalents of an amine in which n is 1, 2, 3 or 4 depending upon the number of carboxyl groups in the carboxylic acid moiety i ), characterised in that the amine ii) has the formula:

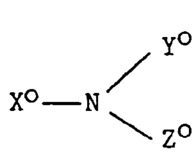

in which X° is $C_{10}$-$C_{24}$ unsubstituted alkyl or $C_2$-$C_{24}$ alkyl interrupted by one or more oxygen atoms; and Y° and Z°, independently, are hydrogen, $C_1$-$C_{24}$ unsubstituted alkyl or $C_2$-$C_{24}$ alkyl interrupted by one or more oxygen atoms or substituted by hydroxy, or are phenyl or $C_7$-$C_9$ phenylalkyl.

8. Salts according to clam 7 wherein one of the substituents R is hydrogen, $C_1$-$C_4$ alkyl or $C_1$-$C_4$ alkoxy and the other R are hydrogen.

9. Salts according to claim 8 wherein all four substituents R are hydrogen.

10. Salts according to claim 7 wherein X° is $C_{10}$-$C_{18}$ alkyl and Y° and Z° are hydrogen or $C_1$-$C_{18}$ alkyl.

**11.** Bis(t-tridecylammonium)-benzothiazol-2-ylthiosuccinate according to claim 7.

**12.** Bis(isononyloxypropylammonium)-benzothiazol-2-ylthiosuccinate according to claim 7.

## Patentansprüche

**1.** Korrosionsinhibierende Überzugszusamnensetzung, enthaltend
a) einen säureempfindlichen Filmbildner und
b) als Korrosionsinhibitor eine wirksame Menge eines Salzes von
i) einer aliphatischen oder cycloaliphatischen Mono-, Di-, Tri- oder Tetra-carbonsäure, die an ihrem aliphatischen oder cycloaliphatischen Rest durch zumindest eine Gruppe der Formel

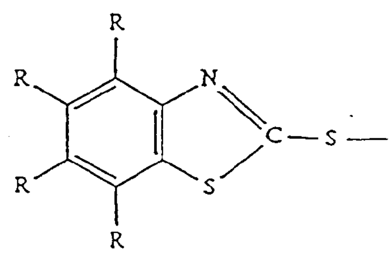

I

substituiert ist, worin jedes R unabhängig von den anderen für Wasserstoff, Alkyl, Halogenoalkyl, Alkoxy, Alkylthio, Alkylsulfonyl, Cycloalkyl, Phenyl, Alkylphenyl, Phenylalkyl, Halogen, -CN, $-NO_2$, -COOH, -COOalkyl, -OH oder eine primäre, sekundäre oder tertiäre Amino- oder Carbamoylgruppe steht;
mit
ii) n molaren Äquivalenten eines Amins, worin n für 1, 2, 3 oder 4 steht in Abhängigkeit von der Anzahl der Carboxylgruppen in der Carbonsäure bi), dadurch gekennzeichnet, daß das Amin bii) die Formel

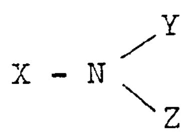

besitzt, worin X unsubstituiertes $C_{10-24}$-Alkyl oder durch ein oder mehrere Sauerstoffatome unterbrochenes $C_{2-24}$-Alkyl ist und Y und Z gleich oder verschieden sind und jeweils Wasserstoff, gegebenenfalls durch ein oder mehrere Sauerstoffatome unterbrochenes Alkyl oder durch Hydroxy substituiertes Alkyl bedeuten oder Phenyl, Phenylalkyl oder Alkylphenyl sind.

**2.** Zusammensetzung gemäß Anspruch 1, worin einer der Substituenten R für Wasserstoff, $C_{1-4}$-Alkyl oder $C_{1-4}$-Alkoxy steht und die anderen drei Substituenten R Wasserstoff sind.

**3.** Zusammensetzung gemäß Anspruch 2, worin alle vier Substituenten R Wasserstoff bedeuten.

**4.** Zusammensetzung gemäß Anspruch 1, worin die Komponente bi) eine Verbindung der Formel II

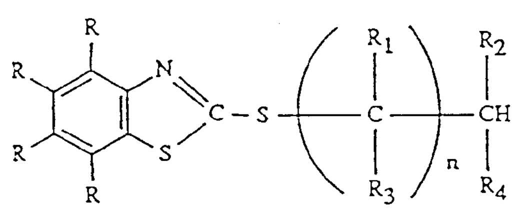

II

ist, worin R wie in Anspruch 1 definiert ist, n für 0 oder 1 steht und $R_1$, $R_2$, $R_3$ und $R_4$ unabhängig vonein-

ander Wasserstoff, Alkyl, Hydroxyalkyl, Halogenoalkyl, Alkoxyalkyl, Carboxyalkyl, Carboxyl oder Phenyl oder Phenylalkyl, welches unsubstituiert, monosubstituiert oder disubstituiert ist, bedeuten oder $R_1$ und $R_2$ oder $R_1$ und $R_3$ gemeinsam lineares oder verzweigtes Alkylen bedeuten, das durch eine oder zwei Carboxylgruppen substituiert sein kann, oder $R_1$ und $R_2$ gemeinsam eine direkte Bindung darstellen und zumindest einer der Substituenten $R_1$, $R_2$, $R_3$ und $R_4$ eine Carboxyl- oder Carboxyalkylgruppe ist.

**5.** Zusammensetzung gemäß Anspruch 4, worin n für 1 steht.

**6.** Zusammensetzung gemäß Anspruch 4, worin $R_4$ eine Carboxylgruppe bedeutet.

**7.** Salze von i) einer aliphatischen oder cycloaliphatischen Mono-, Di-, Tri- oder Tetra-carbonsäure, die an ihrem aliphatischen oder cycloaliphatischen Rest durch zumindest eine Gruppe der Formel

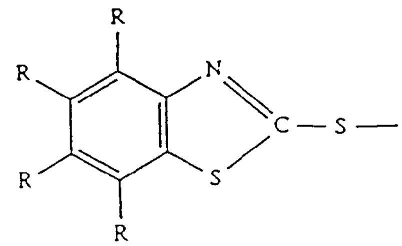

I

substituiert ist, worin jedes R unabhängig von den anderen für Wasserstoff, Alkyl, Halogenalkyl, Alkoxy, Alkylthio, Alkylsulfonyl, Cycloalkyl, Phenyl, Alkylphenyl, Phenylalkyl, Halogen, -CN, $-NO_2$, -COOH, COOalkyl, -OH oder eine primäre, sekundäre oder tertiäre Amino- oder Carbamoylgruppe steht; mit ii) n molaren Äquivalenten eines Amins, worin n für 1, 2, 3 oder 4 in Abhängigkeit von der Anzahl der Carboxylgruppen in dem Carbonsäureteil i) steht, dadurch gekennzeichnet, daß das Amin ii) die Formel

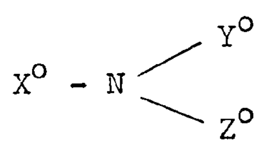

besitzt, worin $X^o$ unsubstituiertes $C_{10-24}$-Alkyl oder durch ein oder mehrere Sauerstoffatom unterbrochenes $C_{2-24}$-Alkyl ist; und $Y^o$ und $Z^o$ unabhängig Wasserstoff, unsubstituiertes $C_{1-24}$-Alkyl oder durch ein oder mehrere Sauerstoffatome unterbrochenes oder durch Hydroxy substituiertes $C_{2-24}$Alkyl bedeuten oder Phenyl oder $C_{7-9}$-Phenylalkyl sind.

**8.** Salze gemäß Anspruch 7, worin einer der Substituenten R für Wasserstoff, $C_{1-4}$-Alkyl oder $C_{1-4}$-Alkoxy steht und die anderen Substituenten R Wasserstoff sind.

**9.** Salze gemäß Anspruch 8, worin alle vier Substituenten R für Wasserstoff stehen.

**10.** Salze gemäß Anspruch 7, worin $X^o$ $C_{10-18}$-Alkyl bedeutet und $Y^o$ und $Z^o$ für Wasserstoff oder $C_{1-18}$-Alkyl stehen.

**11.** Bis-(tert.-tridecylammonium)-benzothiazol-2-yl-thiosuccinat gemäß Anspruch 7.

**12.** Bis-(isononyloxypropylammonium)-benzothiazol-2-yl-thiosuccinat gemäß Anspruch 7.

## Revendications

**1.** Composition de revêtement inhibitrice de corrosion, comprenant :
a) un agent filmogène sensible aux acides, et
b) comme inhibiteur de corrosion, une quantité efficace d'un sel de

i) un acide mono-, di-, tri- ou tétracarboxylique aliphatique ou cycloaliphatique dont le radical aliphatique ou cycloaliphatique est substitué par au moins un groupe de formule

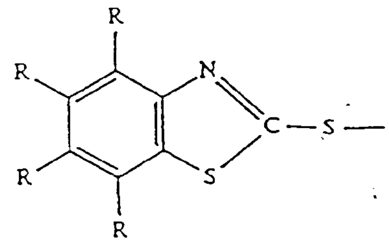

I

où chaque R, indépendamment des autres, est l'hydrogène, un radical alkyle, halogénalkyle, alcoxy, alkylthio, alkylsulfonyle, cycloalkyle, phényle, alkylphényle, phénylalkyle, halogéno, -CN, $-NO_2$, -COOH, -COOalkyle, -OH ou un groupe amino ou carbamyle primaire, secondaire ou tertiaire ; avec

ii) n équivalents molaires d'une amine, n étant 1, 2, 3 ou 4 selon le nombre de groupes carboxyle contenus dans l'acide carboxylique b-i),

caractérisée en ce que l'amine b-ii) répond à la formule :

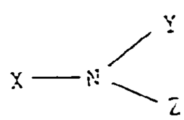

où X est un radical alkyle non substitué en $C_{10}$-$C_{24}$ ou un radical alkyle en $C_2$-$C_{24}$ interrompu par un ou plusieurs atomes d'oxygène, et Y et Z sont identiques ou différents et chacun est l'hydrogène, un radical alkyle facultativement interrompu par un ou plusieurs atomes d'oxygène ou un radical alkyle substitué par un groupe hydroxyle, ou un radical phényle, phénylalkyle ou alkylphényle.

2. Composition selon la revendication 1, dans laquelle l'un des substituants R est l'hydrogène, un radical alkyle en $C_1$-$C_4$ ou un radical alcoxy en $C_1$-$C_4$, et les trois autres sont de l'hydrogène.

3. Composition selon la revendication 2, dans laquelle les quatre substituants R sont tous de l'hydrogène.

4. Une composition selon la revendication I, dans laquelle le composant b-i) est un composé de formule II

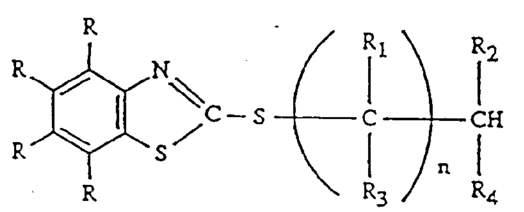

II

où R est tel que défini dans la revendication 1, n est 0 ou 1 et $R_1$, $R_2$, $R_3$ et $R_4$ sont chacun, indépendamment des autres, l'hydrogène, un radical alkyle, hydroxyalkyle, halogénalkyle, alcoxyalkyle, carboxyalkyle, carboxyle ou phényle ou phénylalkyle qui est non substitué ou monosubstitué ou disubstitué, ou bien $R_1$ et $R_2$ ou $R_1$ et $R_3$ représentent ensemble un groupe alkylène linéaire ou ramifié qui peut être substitué par 1 ou 2 groupes carboxyle, ou bien $R_1$ et $R_2$ représentent ensemble une liaison directe, et l'un au moins des substituants $R_1$, $R_2$, $R_3$ et $R_4$ est un groupe carboxyle ou carboxyalkyle.

5. Composition selon la revendication 4, dans laquelle n est 1.

6. Composition selon la revendication 4, dans laquelle $R_4$ est un groupe carboxyle.

7. Sels de i) un acide mono-, di-, tri- ou tétracarboxylique aliphatique ou cycloaliphatique dont le radical aliphatique ou cycloaliphatique est substitué par au moins un groupe de formule

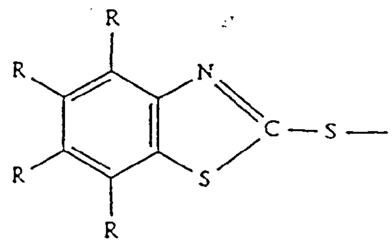

I

où chaque R, indépendamment des autres, est l'hydrogène, un radical alkyle, halogénalkyle, alcoxy, alkylthio, alkylsulfonyle, cycloalkyle, phényle, alkylphényle, phénylalkyle, halogéno, -CN, $-NO_2$, -COOH, -COOalkyle, -OH ou un groupe amino ou carbamyle primaire, secondaire ou tertiaire ; avec ii) n équivalents molaires d'une amine, n étant 1, 2, 3 ou 4 selon le nombre de groupes carboxyle contenus dans la portion acide carboxylique i),
caractérisés en ce que l'amine ii) répond à la formule :

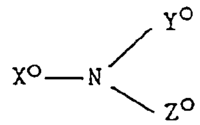

où X° est un radical alkyle non substitué en $C_{10}$-$C_{24}$ ou un radical alkyle en $C_2$-$C_{24}$ interrompu par un ou plusieurs atomes d'oxygène, et Y° et Z°, indépendamment, sont chacun l'hydrogène, un radical alkyle non substitué en $C_1$-$C_{24}$ ou un radical alkyle en $C_2$-$C_{24}$ interrompu par un ou plusieurs atomes d'oxygène ou substitué par un groupe hydroxyle, ou un radical phényle ou phénylalkyle en $C_7$-$C_9$.

**8.** Sels selon la revendication 7, dans lesquels l'un des substituants R est l'hydrogène, un radical alkyle en $C_1$-$C_4$ ou alcoxy en $C_1$-$C_4$ et les autres R sont de l'hydrogène.

**9.** Sels selon la revendication 8, dans lesquels les quatre substituants R sont tous de l'hydrogène.

**10.** Sels selon la revendication 7, dans lesquels X° est un radical alkyle en $C_{10}$-$C_{18}$ et Y° et Z° sont chacun l'hydrogène ou un radical alkyle en $C_1$-$C_{18}$.

**11.** Benzothiazole-2-ylthiosuccinate de bis(*t*-tridécylammonium) selon la revendication 7.

**12.** Benzothiazole-2-ylthiosuccinate de bis(isononyloxypropylammonium) selon la revendication 7.